# EUROPEAN PATENT APPLICATION

(11) **EP 2 221 297 A1**
(43) Date of publication of application: **25.08.2010**
(21) Application number: 09382021.5
(22) Date of filing: 18.02.2009
(51) Int. Cl.: C07D 215/26, A61K 31/4704, A61P 11/06

(54) **5-(2-{[6-(2,2-difluoro-2-phenylethoxy)hexyl]amino}-1-hydroxyethyl)-8-hydroxyquinolin-2(1h)-one and its use in the treatment of pulmonary diseases**

(71) Applicant: Almirall, S.A., 08022 Barcelona (ES)
(72) Inventor: Ruf, Thorsten, 08950, ESPLUGUES DE LLOBREGAT (ES); Massana Montejo, Eric, 08006, BARCELONA (ES)
(74) Representative: Elzaburu Marquez, Alberto

(57) **Abstract**

The present invention provides a compound which is a hydroxyquinolinone derivative of formula (I), 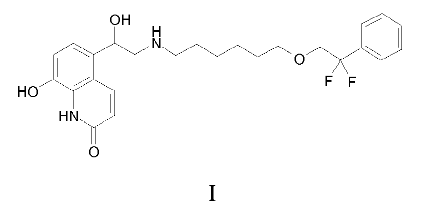 in the form of a racemate, a stereoisomer or a mixture of stereoisomers, or a pharmaceutically acceptable salt or solvate thereof, for use in a method of treating a pulmonary disease or condition associated with β2 adrenergic receptor activity in a mammal, in which the compound is administered by the inhalatory route at a metered nominal dose of less than 5 µg.

## Description

### Field of the Invention

The invention relates to novel methods of beta agonist therapy, particularly for the treatment of respiratory diseases such as asthma and chronic obstructive pulmonary disease (COPD) in a mammal, whilst minimising systemic beta agonist effects. Also provided are pharmaceutical compositions and inhalers that are suitable for use in said novel methods.

### Background of the Invention

5-(2-{[6-(2,2-difluoro-2-phenylethoxy)hexyl]amino}-1-hydroxyethyl)-8-hydroxyquinolin-2(1H)-one is a potent, long acting, selective β2 adrenergic receptor agonist and is described in WO 2006/122788. It has the structure shown below.

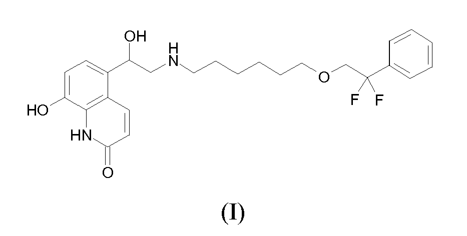

Salt forms of this compound are described in WO 2008/095720.

Currently available long acting β2 adrenergic receptor agonists include salmeterol (2-(hydroxymethyl)-4-{1-hydroxy-2-[6-(4-phenylbutoxy) hexylamino]ethyl}phenol) and formoterol (N-[2-hydroxy-5-[1-hydroxy-2-[1-(4-methoxyphenyl) propan-2-ylamino]ethyl] phenyl]formamide).

Adrenaline is a hormone and neurotransmitter chiefly associated with the sympathetic nervous system. It helps control the functioning of the heart, blood vessels, airways and organs of the digestive tract. Adrenergic agonists mimic the effect of adrenalin at adrenergic receptors. Adrenergic receptors are divided into five categories: α1, α2, β1, β2, and β3. Compounds that mimic adrenaline at the β2 adrenergic receptors are useful in the treatment of respiratory diseases, as they cause smooth muscle contraction, resulting in dilation of the bronchial passages.

One problem with the use of β2 adrenergic agonists in the treatment of respiratory diseases, however, is the risk of side effects related to systemic β adrenergic receptor agonism. These can include, for example, increased heart rate (tachycardia), palpitations insomnia, anxiety, nausea and tremor. Increased heart rate and palpitations can be particularly dangerous in patients suffering from a pre-existing heart condition or a condition that would be aggravated by tachycardia, for example atherosclerosis, restenosis or plaque in the coronary arteries, propensity to arrhythmias, angina pectoris, myocardial infarction, damage resulting from prior heart attacks and congestive heart failure.

Accordingly, there is a need for β2 adrenergic agonist therapy, particularly for respiratory diseases, especially asthma and COPD, which minimises the harmful, systemic side-effects commonly associated with β2 adrenergic agonists.

### Summary of the Invention

It has now been discovered that 5-(2-{[6-(2,2-difluoro-2-phenylethoxy) hexyl]amino}-1-hydroxyethyl)-8-hydroxyquinolin-2(1H)-one has a large therapeutic window and that a maximal therapeutic effect in the lungs can be achieved with even very low doses. In particular, it has been found that a low dosage allows systemic adrenergic agonism side-effects to be minimised, without sacrificing potency of the medicament.

Accordingly, the invention provides, in a first embodiment, a compound which is 5-(2-{[6-(2,2-difluoro-2-phenylethoxy)hexyl]amino}-1-hydroxyethyl)-8-hydroxyquinolin-2(1H)-one in the form of a racemate, a steroisomer or a mixture of stereoisomers, or a pharmaceutically acceptable salt or solvate thereof, for use in a method of treating a pulmonary disease or condition associated with β2 adrenergic receptor activity in a mammal, in which the compound is administered by the inhalatory route at a metered nominal dose of less than 5 µg per inhalation.

As mentioned above, patients suffering from pre-existing heart conditions or conditions that would be aggravated by tachycardia are at particular risk from systemic beta agonist side effects, such as tachycardia.

Accordingly, the invention provides in a second embodiment, a compound which is 5-(2-{[6-(2,2-difluoro-2-phenylethoxy)hexyl]amino}-1-hydroxyethyl)-8-hydroxyquinolin-2(1H)-one, or a pharmaceutically acceptable salt or solvate thereof as defined herein, for use in a method of treating a pulmonary disease or condition associated with β2 adrenergic receptor activity as defined herein, in a mammal as defined herein, without producing in said mammal systemic adrenergic effects.

A third embodiment of the invention is a compound which is 5-(2-{[6-(2,2-difluoro-2-phenylethoxy)hexyl]amino}-1-hydroxyethyl)-8-hydroxyquinolin-2(1H)-one, or a pharmaceutically acceptable salt or solvate thereof as defined herein, for use in a method of treating a pulmonary disease or condition associated with β2 adrenergic receptor activity as defined herein, in a mammal as defined herein, wherein the mammal is suffering from a pre-existing heart condition or a condition that would be aggravated by tachycardia.

Also provided are novel pharmaceutical compositions and inhalers containing the compound of the invention in specific amounts.

### Detailed description of the invention

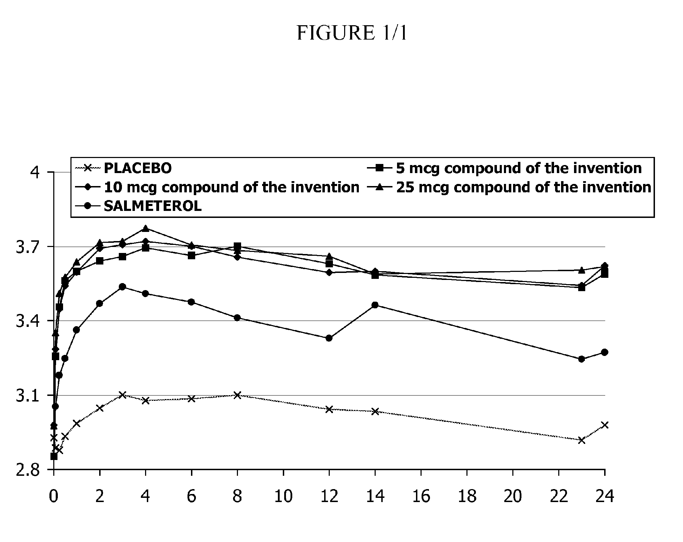

Figure 1 shows the mean FEV1 values (litres) over time (hours) for single doses (5, 10 and 25 µg) of 5-(2-{[6-(2,2-difluoro-2-phenylethoxy)hexyl]amino}-1-(R)-hydroxyethyl)-8-hydroxyquinolin-2(1H)-one referred to in the figure as "Compound 1".

It also shows mean FEV1 values following two administrations, at time points 0 and 12 hours, of 50 µg of salmeterol and a single administration of placebo.

The term "therapeutically effective amount" refers to an amount sufficient to effect treatment when administered to a patient in need of treatment.

The term "metered nominal dose" refers to the quantity of drug substance contained in the metering chamber of the delivery device and is normally expressed as quantity per inhalation. Upon actuation, the drug substance leaves the device and becomes available to the patient as a so-called "emitted dose", which is normally smaller than the metered nominal dose, due to the mechanics of the device.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "pulmonary disease or condition associated with β2 adrenergic receptor activity" includes all pulmonary disease states and/or conditions that are acknowledged now, or that are found in the future, to be associated with β2 adrenergic receptor activity. Such disease states include, but are not limited to asthma and chronic obstructive pulmonary disease (including chronic bronchitis and emphysema). Preferred pulmonary diseases or conditions associated with β2 adrenergic receptor activity are asthma and COPD.

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Salts derived from pharmaceutically-acceptable acids include acetic, benzenesulfonic, benzoic, camphosulfonic, citric, ethanesulfonic, fumaric, gluconic, glutamic, hydrobromic, hydrochloric, lactic, maleic, malic, mandelic, methanesulfonic, mucic, naphthalene-1,5-disulphonic acid (napadisylate), nitric, pantothenic, phosphoric, succinic, sulfuric, tartaric, p-toluenesulfonic, xinafoic (1-hydroxy-2-naphthoic acid) and the like. Particularly preferred are salts derived from fumaric, hydrobromic, hydrochloric, acetic, sulfuric, methanesulfonic, naphthalene-1,5-disulphonic, xinafoic, and tartaric acids. Most preferred are salts derived from methanesulfonic and naphthalene-1,5-disulphonic acids.

Salts derived from naphthalene-1,5-disulphonic acid are typically mononapadisylate or heminapadisylate salts and pharmaceutically acceptable solvates thereof.

Typically, naphthalene-1,5-disulfonic acid salts of compounds of the invention have the formula (II): 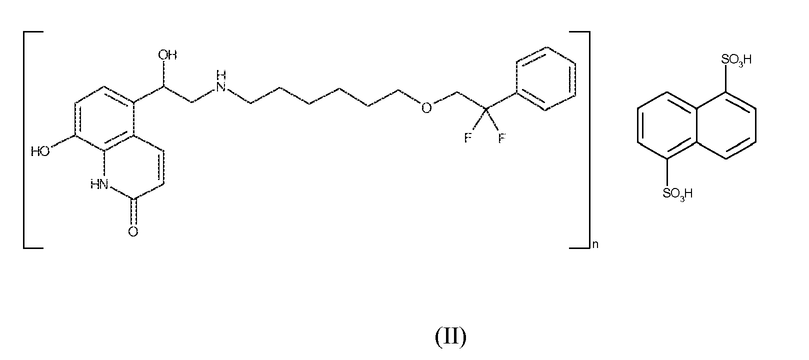 wherein n has a value of 1 or 2.

Salts derived from methanesulphonic acid are also known as mesylates

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

The term "solvate" refers to a complex or aggregate formed by one or more molecules of a solute, i.e. a compound of the invention or a pharmaceutically-acceptable salt thereof, and one or more molecules of a solvent. Such solvates are typically crystalline solids having a substantially fixed molar ratio of solute and solvent. Representative solvents include by way of example, water, methanol, ethanol, isopropanol, acetic acid, and the like. When the solvent is water, the solvate formed is a hydrate.

The compound of the invention contains a chiral center. Accordingly, it can be used in the form of a racemic mixture, an enantiomer, or a mixture enriched in one or more stereoisomer. The scope of the invention as described and claimed encompasses the racemic form of the compound of the invention as well as the individual enantiomers, and stereoisomer-enriched mixtures.

The most preferred enantiomer is 5-(2-{[6-(2,2-difluoro-2-phenylethoxy)hexyl]amino}-1-(R)-hydroxyethyl)-8-hydroxyquinolin-2(1H)-one.

It will be appreciated that the term "or a pharmaceutically acceptable salt or solvate thereof" is intended to include all permutations of salts and solvates, such as a solvate of a pharmaceutically-acceptable salt of a compound of the invention.

In the present invention, a metered nominal dose per inhalation of less than 5µg of a compound of the invention can be administered. It is to be understood, though, that at least some amount of a compound of the invention must be present. The minimum amount of the compound of the invention is therefore greater than 0µg, preferably greater than 0.01µg, more preferably greater than 0.025µg, most preferably greater than 0.05µg.

In preferred embodiments, the compound of the invention is administered at a metered nominal dose per inhalation of less than 4.5µg, less than 4µg, less than 3.5µg, less than 3µg, less than 2.5µg, less than 2µg, less than 1.5µg, less than 1µg, less than 0.5µg, less than 0.25µg, or less than 0.1µg.

Typically, the compound of the invention is administered by inhalation. The compound of the invention is preferably administered by inhalation from an inhaler or nebuliser.

Typically, the compound of the invention is in a pharmaceutical composition comprising any suitable excipients or pharmaceutically acceptable carriers and in the form of a dry powder or a solution suitable for inhalation.

Typically, the compound of the invention is administered at an amount per inhalation equivalent to a metered nominal dose of less than 5 µg of the heminapadysilate salt administered with a dry powder inhaler.

Typically, the compound of the invention is administered as a single-dose treatment or in continued treatments with one or more doses per day, preferably from 1 to 4 doses per day, more preferably from 1 to 2 doses per day, even more preferably as 1 dose per day.

Typically, the pulmonary disease or condition associated with β2 adrenergic receptor activity is asthma or COPD.

Typically, the mammal treated is a human.

Typically, the hydroxyquinolinone derivative of formula (I) is chosen from (R,S) 5-(2-{[6-(2,2-difluoro-2-phenylethoxy)hexyl]amino}-1-hydroxyethyl)-8-hydroxyquinolin-2(1H)-one and 5-(2-{[6-(2,2-difluoro-2-phenylethoxy)hexyl]amino}-1(R)-hydroxyethyl)-8-hydroxyquinolin-2(1H)-one.

Preferably, the hydroxyquinolinone derivative of formula (I) is present as the R-enantiomer.

Alternatively, the hydroxyquinolinone derivative of formula (I) is present as the S-enantiomer.

Typically, the compound of the invention is a pharmaceutically acceptable salt of a hydroxyquinolinone derivative of formula (I) as defined herein. Preferably, the compound of the invention is a mesylate, mononapadisylate or heminapadisylate salt of a hydroxyquinolinone derivative of formula (I) as defined herein.
Preferably, the hydroxyquinolinone derivative of formula (I) is selected from one of 5-(2-{[6-(2,2-difluoro-2-phenylethoxy)hexyl]amino}-1(R)-hydroxy-ethyl)-8-hydroxyquinolin-2(1H)-one heminapadisylate, or a pharmaceutically acceptable solvate thereof, and 5-(2-{[6-(2,2-difluoro-2-phenylethoxy)hexyl]amino}-1(R)-hydroxy-ethyl)-8-hydroxyquinolin-2(1H)-one mesylate, or a pharmaceutically acceptable solvate thereof.

Typically, the compound is co-administered with a therapeutically effective amount of another therapeutic agent.

The other therapeutic agent is typically a corticosteroid, an anticholinergic agent, and/or a PDE4 inhibitor.

Examples of suitable PDE4 inhibitors are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid (MK-0873), CDC-801, UK-500001, BLX-914, 2-carbomethoxy-4-cyano-4-(3-cyclopropylmethoxy-4-difluroromethoxyphenyl)cyclohexanl-one, *cis* [4-cyano-4-(3-cyclopropylmethoxy-4-difluoromethoxyphenyl)cyclohexan-1-ol, CDC-801, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the PCT patent applications number WO03/097613, W02004/058729, WO 2005/049581, WO 2005/123693 and WO 2005/123692.

Examples of suitable corticosteroids and glucocorticoids are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methylsulfanyl)acetoxy]-4-pregnene-3,20-dione, Desisobutyrylciclesonide, hydrocortisone acetate, hydrocortisone sodium succinate, NS-126, prednisolone sodium phosphate and hydrocortisone probutate, Prednisolone sodium metasulfobenzoate and clobetasol propionate

Examples of suitable M3 antagonists (anticholinergics) are tiotropium salts, oxitropium salts, flutropium salts, ipratropium salts, glycopyrronium salts, trospium salts, zamifenacin, revatropate, espatropate, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1(R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl] carbamoyl] ethyl] carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Particularly preferred additional therapeutic agents are selected from the group consisting of mometasone furoate, ciclesonide, budesonide, fluticasone propionate, fluticasone furoate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, rolipram, roflumilast, cilomilast and the compounds claimed in the PCT patent applications number WO03/097613, WO2004/058729, WO 2005/049581, WO 2005/123693 and WO 2005/123692.

Thus, one aspect of the invention provides a compound of the invention as defined herein, and a corticosteroid for simultaneous, concurrent separate or sequential use in a method of treating a pulmonary disease or condition associated with β2 adrenergic receptor activity as defined herein, in a mammal as defined herein, in which the compound of the invention is administered by the inhalatory route at a metered nominal dose of less than 5 µg per inhalation.

Also provided is a compound of the invention for use in a method of treating a pulmonary disease or condition associated with β2 adrenergic receptor activity as defined herein, in a mammal as defined herein, in which the compound of the invention is administered by the inhalatory route at a metered nominal dose of less than 5 µg per inhalation, and in which the compound of the invention is co-administered with a corticosteroid.

Also provided is a corticosteroid for use in a method of treating a pulmonary disease or condition associated with β2 adrenergic receptor activity as defined herein, in a mammal as defined herein, by co-administration with a compound of the invention, wherein the compound of the invention is administered by the inhalatory route at a metered nominal dose of less than 5 µg per inhalation.

Particularly preferred corticosteroids are those selected from the group consisting of mometasone furoate, ciclesonide, budesonide, fluticasone furoate and fluticasone propionate.

Another aspect of the invention provides a compound of the invention as defined herein, and an anticholinergic agent and, optionally, a corticosteroid for simultaneous, concurrent separate or sequential use in a method of treating a pulmonary disease or condition associated with β2 adrenergic receptor activity as defined herein, in a mammal as defined herein, in which the compound of the invention is administered by the inhalatory route at a metered nominal dose of less than 5 µg per inhalation.

Also provided is a compound of the invention as defined herein for use in a method of treating a pulmonary disease or condition associated with β2 adrenergic receptor activity as defined herein, in a mammal as defined herein, in which the compound of the invention is administered by the inhalatory route at a metered nominal dose of less than 5 µg per inhalation, and in which the compound of the invention is co-administered with an anticholinergic agent and, optionally, a corticosteroid.

Also provided is an anticholinergic agent for use in a method of treating a pulmonary disease or condition associated with β2 adrenergic receptor activity as defined herein, in a mammal as defined herein, by co-administration with a compound of the invention and, optionally a corticosteroid, wherein the compound of the invention is administered by the inhalatory route at a metered nominal dose of less than 5 µg per inhalation.

Particularly preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]octane salts. Optional corticosteroids are preferably selected from the group consisting of mometasone furoate, ciclesonide, budesonide, fluticasone furoate and fluticasone.

A still further aspect of the invention provides a compound of the invention as defined herein, and a PDE4 inhibitor and, optionally, a corticosteroid, and/or an anticholinergic agent, for simultaneous, concurrent separate or sequential use in a method of treating a pulmonary disease or condition associated with β2 adrenergic receptor activity as defined herein, in a mammal as defined herein, in which the compound of the invention is administered by the inhalatory route at a metered nominal dose of less than 5 µg per inhalation.

Also provided is a compound of the invention for use in a method of treating a pulmonary disease or condition associated with β2 adrenergic receptor activity as defined herein, in a mammal as defined herein, in which the compound of the invention is administered by the inhalatory route at a metered nominal dose of less than 5 µg per inhalation, and in which the compound of the invention is co-administered with a PDE4 inhibitor and, optionally, a corticosteroid, and/or an anticholinergic agent.

Also provided is a PDE4 inhibitor for use in a method of treating a pulmonary disease or condition associated with β2 adrenergic receptor activity as defined herein, in a mammal as defined herein, by co-administration with a compound of the invention, and, optionally, a corticosteroid, and/or an anticholinergic agent, wherein the compound of the invention is administered by the inhalatory route at a metered nominal dose of less than 5 µg per inhalation.

Particularly preferred PDE4 inhibitors are those selected from the group consisting of rolipram, roflumilast, cilomilast and the compounds claimed in the PCT patent applications number WO03/097613, W02004/058729, WO 2005/049581, WO 2005/123693 and WO 2005/123692. Optional corticosteroids are preferably selected from the group consisting of mometasone furoate, ciclesonide, budesonide, fluticasone furoate and fluticasone propionate. Optional anticholinergic agents are preferably selected from the group consisting oftiotropium salts, glycopirronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

A particularly preferred embodiment of the present invention provides a compound of the invention as defined herein, and a therapeutically effective amount of a 3-[2-Hydroxy-2,2-bis(2-thienyl)acetoxy]-1-(3-phenoxypropyl)-1-azoniabicyclo[2.2.2]octane salt and, optionally, a corticosteroid and/or a PDE4 inhibitor for simultaneous, concurrent, separate or sequential use in a method of treating a pulmonary disease or condition associated with β2 adrenergic receptor activity as defined herein, in a mammal as defined herein, in which the compound of the invention is administered by the inhalatory route at a metered nominal dose of less than 5 µg.

Also provided is a compound of the invention as defined herein for use in a method of treating a pulmonary disease or condition associated with β2 adrenergic receptor activity as defined herein, in a mammal as defined herein, in which the compound of the invention is administered by the inhalatory route at a metered nominal dose of less than 5 µg per inhalation, and in which the compound of the invention is co-administered with a therapeutically effective amount of a 3-[2-Hydroxy-2,2-bis(2-thienyl)acetoxy]-1-(3-phenoxypropyl)-1-azoniabicyclo[2.2.2]octane salt and, optionally, a corticosteroid and/or a PDE4 inhibitor.

Also provided is a therapeutically effective amount of a 3-[2-Hydroxy-2,2-bis(2-thienyl)acetoxy]-1-(3-phenoxypropyl)-1-azoniabicyclo[2.2.2]octane salt for use in a method of treating a pulmonary disease or condition associated with β2 adrenergic receptor activity as defined herein, in a mammal as defined herein, by co-administration with a compound of the invention and, optionally, a corticosteroid and/or a PDE4 inhibitor, wherein the compound of the invention is administered by the inhalatory route at a metered nominal dose of less than 5 µg per inhalation.

Another particularly preferred embodiment of the present invention provides a compound of the invention as defined herein, and a therapeutically effective amount of mometasone furoate and, optionally, an anticholinergic and/or a PDE4 inhibitor for simultaneous, concurrent separate or sequential use in a method of treating a pulmonary disease or condition associated with β2 adrenergic receptor activity as defined herein, in a mammal as defined herein, in which the compound of the invention is administered by the inhalatory route at a metered nominal dose of less than 5 µg per inhalation.

Also provided is a compound of the invention as defined herein for use in a method of treating a pulmonary disease or condition associated with β2 adrenergic receptor activity as defined herein, in a mammal as defined herein, in which the compound of the invention is administered by the inhalatory route at a metered nominal dose of less than 5 µg per inhalation, and in which the compound of the invention is co-administered with a therapeutically effective amount of mometasone furoate and, optionally, an anticholinergic and/or a PDE4 inhibitor.

Also provided is a therapeutically effective amount of mometasone furoate for use in a method of treating a pulmonary disease or condition associated with β2 adrenergic receptor activity as defined herein, in a mammal as defined herein, by co-administration with a compound of the invention and, optionally, an anticholinergic and/or a PDE4 inhibitor, wherein the compound of the invention is administered by the inhalatory route at a metered nominal dose of less than 5 µg per inhalation.

Yet another embodiment of the invention provides a compound of the invention as defined herein, a corticosteroid, an anticholinergic agent and a PDE4 inhibitor for simultaneous, concurrent, separate or sequential use in a method of treating a pulmonary disease or condition associated with β2 adrenergic receptor activity as defined herein, in a mammal as defined herein, in which the compound of the invention is administered by the inhalatory route at a metered nominal dose of less than 5 µg per inhalation.

Typically, administration of the compound of the invention by inhalation does not result in a quantifiable level of the compound of the invention in the blood plasma of the mammal in the time period from 1.5 to 24 hours after administration of the drug, more typically in the time period from 1 to 24 hours after administration of the drug, more typically in the time period from 0.5 to 24 hours after administration of the drug. Preferably, administration of the compound of the invention by inhalation does not result in a quantifiable level of the compound in the blood plasma of the mammal at any time.

As used herein, a quantifiable level is typically an amount equal to or greater than 2.5 pg/ml.

The compound of the invention is advantageous because it avoids one or more systemic beta agonist effects typically associated with the use of beta agonists. Thus, the mammal is typically substantially free of increased heart rate (tachycardia), palpitations, insomnia, anxiety, nausea and/or tremor following the administration of the compound of the invention.

As mentioned above, the invention provides in further embodiments, a compound which is 5-(2-{[6-(2,2-difluoro-2-phenylethoxy)hexyl]amino}-1-hydroxyethyl)-8-hydroxyquinolin-2(1H)-one, or a pharmaceutically acceptable salt or solvate thereof as defined herein, for use in a method of treating a pulmonary disease or condition associated with β2 adrenergic receptor activity as defined herein, in a mammal as defined herein, without producing in said mammal systemic adrenergic effects or wherein the mammal is suffering from a pre-existing heart condition or condition that would be aggravated by tachycardia.

Typically, in these embodiments, the compound is administered by the inhalatory route at a metered nominal dose of less than 5 µg per inhalation.

Typically, in these embodiments, the compound is administered once daily.

Typically, in these embodiments, the compound is coadministered with a therapeutically effective amount of a corticosteroid, an anticholinergic agent, and/or a PDE4 inhibitor.

Typically, in these embodiments, administration of the compound of the invention by inhalation does not result in a quantifiable level of the compound of the invention in the blood plasma of the mammal in the time period from 1.5 to 24 hours after administration of the drug, more typically in the time period from 1 to 24 hours after administration of the drug, more typically in the time period from 0.5 to 24 hours after administration of the drug. Preferably, administration of the compound of the invention by inhalation does not result in a quantifiable level of the compound in the blood plasma of the mammal at any time.

Typically, in these embodiments, the mammal is substantially free of increased heart rate (tachycardia), palpitations, insomnia, anxiety, nausea and/or tremor following the administration of the compound.

The pre-existing heart condition or condition that would be aggravated by tachycardia is typically selected from atherosclerosis, restenosis or plaque in the coronary arteries, propensity to arrhythmias, angina pectoris, myocardial infarction, damage resulting from prior heart attacks and congestive heart failure.

Typically, the pharmaceutical compositions comprising a compound of the present invention and a pharmaceutically acceptable carrier are suitable for administration by inhalation and may further comprise a therapeutically effective amount of one or more other therapeutic agents, as defined herein. However, any other form of topical, parenteral or oral application is possible. The application of inhaled dosage forms embodies the preferred application form, especially in the therapy of diseases or disorders of the lung.

The pharmaceutical compositions may be prepared by any of the methods well known in the art of pharmacy. All methods include the step of bringing the active ingredient(s) into association with the carrier. In general the pharmaceutical compositions are prepared by uniformly and intimately bringing into association the active ingredient(s) with liquid carriers or finely divided solid carriers or both and then, if necessary, shaping the product into the desired form.

The carrier for a pharmaceutical composition in the form of a dry powder is typically chosen from starch or a pharmaceutically acceptable sugar, such as lactose or glucose. Lactose is preferred.

Additional suitable carriers can be found in Remington: The Science and Practice of Pharmacy, 20th Edition, Lippincott Williams & Wilkins, Philadelphia, Pa., 2000.

The pharmaceutical compositions for inhalation are delivered with the help of inhalers, such as dry powder inhalers, aerosols or nebulisers. The inhaler is typically configured to deliver, upon actuation, a therapeutically effective amount of one or more other therapeutic agents, as defined herein.

Packaging of the compound of the invention in the inhaler may be suitable for unit dose or multi-dose delivery. In the case of multi-dose delivery, the compound of the invention can be pre-metered or metered in use.

The inhaler is typically a single dose inhaler, a multiple unit dose inhaler, or a multi dose device inhaler.

Dry powder compositions for topical delivery to the lung by inhalation may, for example, be presented in capsules and cartridges of for example gelatine or blisters of for example laminated aluminium foil, for use in an inhaler or insufflator.

Dry powder inhalers are classified into three groups: (a) single dose, (b) multiple unit dose and (c) multi dose devices.

For inhalers of the first type (a), single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e.g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers (b) together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose devices (c) do not contain pre-measured quantities of the medicament containing powder. They consist of a relatively large container and a dose measuring principle that has to be operated by the patient. The container bears multiple doses that are isolated individually from the bulk of powder by volumetric displacement. Various dose measuring principles exist, including rotatable membranes (e.g. EP0069715) or disks (e.g. GB 2041763; EP 0424790; DE 4239402 and EP 0674533), rotatable cylinders (e.g. EP 0166294; GB 2165159 and WO 92/09322) and rotatable frustums (e.g. WO 92/00771), all having cavities which have to be filled with powder from the container. Other multi dose devices have measuring plungers with a local or circumferential recess to displace a certain volume of powder from the container to a delivery chamber or an air conduit (e.g. EP 0505321, WO 92/04068 and WO 92/04928), or measuring slides such as the Novolizer SD2FL (ex. Sofotec), also known as Genuair®, which is described in the following patent applications: WO 97/000703, WO 03/000325 and WO 03/061742.

Reproducible dose measuring is one of the major concerns for multi dose devices.
The powder formulation has to exhibit good and stable flow properties, because filling of the dose measuring cups or cavities is mostly under the influence of the force of gravity. For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (e.g. WO 97/000703). For such semi-permanent multi dose inhalers with disposable drug containers, the requirements to prevent drug accumulation are even more strict.

Medicaments for administration by inhalation desirably have a controlled particle size. The optimum particle size for inhalation into the bronchial system is usually 1-10µm, preferably 2-5µm. Particles having a size above 20µm are generally too large when inhaled to reach the small airways. To achieve these particle sizes, the particles of the active ingredient as produced may be size reduced by conventional means e.g. by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.

Apart from applications through dry powder inhalers the compositions of the invention can also be administered in nebulisers, metered dose inhalers and aerosols which operate via propellant gases or by means of so-called atomisers, via which solutions of pharmacologically-active substances can be sprayed under high pressure so that a mist of inhalable particles results. Such atomisers are described, for example, in W0 91/14468 and WO 97/12687.

These liquid formulations generally contain a suitable carrier which may be either a propellant for MDI administration or water for administration through a nebuliser. The formulation may comprise additional components such as preservatives (for example, benzalkonium chloride, potassium sorbate, benzyl alcohol); pH stabilizers (for example, acidic agents, alkaline agents, buffer systems); isotonic stabilizers (for example, sodium chloride); surfactant and wetting agents (for example, polysorbates, sorbitan esters); and/or absorption enhancers (for example, chitosan, hyaluronic acid, surfactants). The formulation may also contain additives to improve the solubility of other active compounds when mixed with the salt of the invention. The solubility enhancers may comprise components such as cyclodextrins, liposomes or co-solvents such as ethanol, glycerol and propylene glycol.

Additional suitable carriers for formulations of the active salts of the present invention can be found in Remington: The Science and Practice of Pharmacy, 20th Edition, Lippincott Williams & Wilkins, Philadelphia, Pa., 2000.

Pressurized aerosol compositions will generally be filled into canisters fitted with a valve, especially a metering valve. Canisters may optionally be coated with a plastics material e.g. a fluorocarbon polymer as described in W096/32150. Canisters will be fitted into an actuator adapted for buccal delivery.

Thus, the present invention also provides a pharmaceutical composition for inhalation comprising 5-(2-{[6-(2,2-difluoro-2-phenylethoxy)hexyl]amino}-1-hydroxyethyl)-8-hydroxyquinolin-2(1H)-one, or a pharmaceutically acceptable salt or solvate thereof as defined herein, and a pharmaceutically acceptable carrier and providing a metered nominal dose of less than 5 µg per inhalation.

The present invention also provides a method of treating a pulmonary disease or condition associated with β2 adrenergic receptor activity, as defined herein, in a mammal, as defined herein, which method comprises administering to said mammal by the inhalatory route a metered nominal dose of less than 5µg of a compound, which is 5-(2-{[6-(2,2-difluoro-2-phenylethoxy)hexyl]amino}-1-hydroxyethyl)-8-hydroxyquinolin-2(1H)-one, or a pharmaceutically acceptable salt or solvate thereof as defined herein.

Typically, in this method, the compound is administered once daily.

Typically, in this method, the compound is coadministered with a therapeutically effective amount of a corticosteroid and/or an anticholinergic agent, and/or a PDE4 inhibitor.

Typically, in this method, administration of the compound of the invention by inhalation does not result in a quantifiable level of the compound of the invention in the blood plasma of the mammal in the time period from 1.5 to 24 hours after administration of the drug, more typically in the time period from 1 to 24 hours after administration of the drug, more typically in the time period from 0.5 to 24 hours after administration of the drug. Preferably, administration of the compound of the invention by inhalation does not result in a quantifiable level of the compound in the blood plasma of the mammal at any time.

Typically, in this method, the mammal is substantially free of increased heart rate (tachycardia), palpitations, insomnia, anxiety, nausea and/or tremor following the administration of the compound.

The invention also provides a method of treating a pulmonary disease or condition associated with β2 adrenergic receptor activity, as defined herein, in a mammal, as defined herein, which method comprises administering to said mammal a therapeutically effective amount of a compound which is 5-(2-{[6-(2,2-difluoro-2-phenylethoxy)hexyl]amino}-1-hydroxyethyl)-8-hydroxyquinolin-2(1H)-one, or a pharmaceutically acceptable salt or solvate thereof as defined herein without producing in said mammal systemic adrenergic effects or wherein the mammal is suffering from a pre-existing heart condition or condition that would be aggravated by tachycardia as defined herein.

Typically, in this method, the compound is administered by the inhalatory route at a metered nominal dose of less than 5 µg per inhalation.

Typically, in this method, the compound is administered once daily.

Typically, in this method, the compound is coadministered with a therapeutically effective amount of a corticosteroid, and/or an anticholinergic agent, and/or a PDE4 inhibitor.

Typically, in this method, administration of the compound of the invention by inhalation does not result in a quantifiable level of the compound of the invention in the blood plasma of the mammal in the time period from 1.5 to 24 hours after administration of the drug, more typically in the time period from 1 to 24 hours after administration of the drug, more typically in the time period from 0.5 to 24 hours after administration of the drug. Preferably, administration of the compound of the invention by inhalation does not result in a quantifiable level of the compound in the blood plasma of the mammal at any time.

Typically, in this method, the mammal is substantially free of increased heart rate (tachycardia), palpitations, insomnia, anxiety, nausea and/or tremor following the administration of the compound.

The present invention also provides use of a compound of the invention, in the manufacture of a medicament for the treatment of a pulmonary disease or condition associated with β2 adrenergic receptor activity, as defined herein, in a mammal, as defined herein, wherein the medicament comprises less than 5µg of a compound of the invention.

The present invention also provides use of a compound of the invention, in the manufacture of a medicament for the treatment of a pulmonary disease or condition associated with β2 adrenergic receptor activity, as defined herein, in a mammal, as defined herein, without producing in said mammal systemic adrenergic effects or wherein the mammal is suffering from a pre-existing heart condition or condition that would be aggravated by tachycardia as defined herein.

### Example 1

Clinical Phase II study: A randomised double-blind, double-dummy, placebo and active comparator-controlled, cross-over trial assesses the activity, safety, tolerability and pharmacokinetics of single doses of 5-(2-{[6-(2,2-difluoro-2-phenylethoxy)hexyl]amino}-1-(R)-hydroxyethyl)-8-hydroxyquinolin-2(1H)-one heminapadisylate by inhalation in asthma patients.

Methods: Men with a diagnosis of mild to moderate persistent asthma, as defined by the 2006 GINA guideline, for at least 6 months prior to screening and with a FEV1 61-85% of the predicted normal values (according to Quanjer et al. 1993) were randomised to treatment sequences comprising a single-dose administration of 5-(2-{[6-(2,2-difluoro-2-phenylethoxy)hexyl]amino}-1-(R)-hydroxyethyl)-8-hydroxyquinolin-2(1H)-one heminapadisylate (at metered nominal doses of 5, 10 and 25 micrograms in the Cyclohaler® device), two administrations (at time points 0 and 12 hours) of Salmeterol (at a metered nominal dose of 50 micrograms in the Cyclohaler® device) and placebo administered all of them by means of a dry powder inhaler. Lung function measurements included (by spirometry) FEV1, PEF, FVC, FEF25-75, and (by body plethysmography) sGaw and Raw.

Results: 25 males (aged 18 to 70) took part in the study. 5-(2-{[6-(2,2-difluoro-2-phenylethoxy)hexyl]ammo}-1-(R)-hydroxyethyl)-8-hydroxyquinolin-2(1H)-one clearly increased the average peak and trough FEV1 compared with placebo and with salmeterol at all the doses tested. Strikingly, the increase in average trough FEV1 is statistically significant compared to salmeterol, even though this compound was administered in two doses. These data are shown as Figure 1.

All (5, 10 and 25 micrograms) doses of 5-(2-{[6-(2,2-difluoro-2-phenylethoxy)hexyl]amino}-1-(R)-hydroxyethyl)-8-hydroxyquinolin-2(1H)-one showed bronchodilatory action up to 36 hours after dosing. There was no evidence of differences in dose effect between the different doses of 5-(2-{[6-(2,2-difluoro-2-phenylethoxy)hexyl]amino}-1-(R)-hydroxyethyl)-8-hydroxyquinolin-2(1H)-one. Similar trends are seen with PEF, FVC, FEF25-75, sGaw and Raw. No plasma level of 5-(2-{[6-(2,2-difluoro-2-phenylethoxy)hexyl]amino}-1-(R)-hydroxyethyl)-8-hydroxyquinolin-2(1H)-one was quantifiable in the plasma samples of subjects receiving the dose of 5 micrograms (LLOQ 2.5pg/ml). Adverse events commonly seen after adrenergic simulation, such as tremor, tachycardia and restlessness, occurred only with the two higher (10 and 25 micrograms) doses of 5-(2-{[6-(2,2-difluoro-2-phenylethoxy)hexyl]amino}-1-(R)-hydroxyethyl)-8-hydroxyquinolin-2(1H)-one tested.

Conclusion: A single dose of 5-(2-{[6-(2,2-difluoro-2-phenylethoxy)hexyl]amino}-1-(R)-hydroxyethyl)-8-hydroxyquinolin-2(1H)-one (5 micrograms) produces a supramaximal long-lasting bronchodilation in patients with asthma without producing systemic adrenergic effects. This large therapeutic window guarantees that this compound can be safely used at lower doses which will not cause adverse effects while still producing a remarkable therapeutic bronchodilatory effect.

## Claims

1. A compound which is 5-(2-{[6-(2,2-difluoro-2-phenylethoxy)hexyl]amino}-1-hydroxyethyl)-8-hydroxyquinolin-2(1H)-one in the form of a racemate, a steroisomer or a mixture of stereoisomers, or a pharmaceutically acceptable salt or solvate thereof, for use in a method of treating a pulmonary disease or condition associated with β2 adrenergic receptor activity in a mammal, in which the compound is administered by the inhalatory route at a metered nominal dose per inhalation of less than 5 µg.

2. A compound according to claim 1, which is administered once daily.

3. A compound according to any one of the preceding claims, wherein the pulmonary disease or condition associated with β2 adrenergic receptor activity is asthma or COPD.

4. A compound according to any one of the preceding claims, wherein the mammal is a human.

5. A compound according to any one of the preceding claims, wherein 5-(2-{[6-(2,2-difluoro-2-phenylethoxy)hexyl]amino}-1-hydroxyethyl)-8-hydroxyquinolin-2(1H)-one is present as the R-enantiomer.

6. A compound according to any one of the preceding claims, wherein the pharmaceutically acceptable salt is a mesylate, mononapadisylate or heminapadisylate salt.

7. A compound according to any one of the preceding claims, which is co-administered with a therapeutically effective amount of a corticosteroid, an anticholinergic agent, and/or a PDE4 inhibitor.

8. A compound according to any of the preceding claims, wherein administration of the compound does not result in a quantifiable level of the compound in the blood plasma of the mammal.

9. A compound according to any one of the preceding claims, wherein the mammal is substantially free of increased heart rate (tachycardia), palpitations, insomnia, anxiety, nausea and/or tremor following the administration of the compound.

10. A compound as defined in any one of claims 1, 5 and 6 for use in a method of treating a pulmonary disease or condition associated with β2 adrenergic receptor activity as defined in any one of claims 1 and 3, in a mammal as defined in any one of claims 1 and 4, without producing systemic adrenergic effects in said mammal.

11. A compound as defined in any one of claims 1, 5 and 6 for use in a method of treating a pulmonary disease or condition associated with β2 adrenergic receptor activity as defined in any one of claims 1 and 3, in a mammal as defined in any one of claims 1 and 4, wherein the mammal is suffering from a pre-existing heart condition or condition that would be aggravated by tachycardia.

12. A compound according to claims 10 or 11, which is administered by the inhalatory route at a meterd nominal dose per inhalation of less than 5 µg, and/or which is administered once daily, and/or which is coadministered with a therapeutically effective amount of a corticosteroid, and/or an anticholinergic agent, and/or a PDE4 inhibitor, and/or wherein administration of the compound by inhalation does not result in a quantifiable level of the compound in the blood plasma of the mammal.

13. A compound according to claims 11 or 12, wherein the pre-existing heart condition or condition that would be aggravated by tachycardia is selected from atherosclerosis, restenosis or plaque in the coronary arteries, propensity to arrhythmias, angina pectoris, myocardial infarction, damage resulting from prior heart attacks and congestive heart failure.

14. A pharmaceutical composition for inhalation comprising a compound as defined in any one of claims 1, 5 or 6 and a pharmaceutically acceptable carrier and providing a metered nominal dose of less than 5 µg per inhalation.

15. A pharmaceutical composition according to claim 14, which further comprises a therapeutically effective amount of one or more other therapeutic agents, as defined in claim 7.

16. An inhaler configured to deliver, upon actuation, a metered nominal dose of less than 5 µg of a compound as defined in any one of claims 1, 5 or 6.
